Europäisches Patentamt

(19) European Patent Office

Office européen des brevets

(11) EP 0 710 086 B1

(12) FASCICULE DE BREVET EUROPEEN

(45) Date de publication et mention
de la délivrance du brevet:
**08.09.1999 Bulletin 1999/36**

(21) Numéro de dépôt: **94922303.6**

(22) Date de dépôt: **25.07.1994**

(51) Int. Cl.6: **A61B 5/05**

(86) Numéro de dépôt international:
**PCT/FR94/00930**

(87) Numéro de publication internationale:
**WO 95/02991 (02.02.1995 Gazette 1995/06)**

(54) **APPAREIL DE MESURE ET DE TRAITEMENT DE SIGNAUX PHYSIOLOGIQUES ET PROCEDE AUTOMATIQUE MIS EN OEUVRE PAR LEDIT APPAREIL**

VORRICHTUNG UND VERFAHREN ZUM MESSEN UND VERARBEITEN VON PHYSIOLOGISCHEN SIGNALEN

APPARATUS FOR MEASURING AND PROCESSING PHYSIOLOGICAL SIGNALS AND AUTOMATIC METHOD THEREFOR

(84) Etats contractants désignés:
AT BE CH DE DK ES FR GB GR IE IT LI LU MC NL PT SE

(30) Priorité: **23.07.1993 FR 9309262**

(43) Date de publication de la demande:
**08.05.1996 Bulletin 1996/19**

(73) Titulaires:
• **Bour, Jean**
**F-57730 Petit-Ebersviller (FR)**
• **Bour, Frank**
**F-57730 Petit-Ebersviller (FR)**

(72) Inventeurs:
• **BOUR, Jean**
**F-57730 Petit-Ebersviller (FR)**
• **BOUR, Frank**
**F-57730 Petit-Ebersviller (FR)**
• **SCHANG, Daniel**
**F-57730 Folschviller (FR)**
• **NOTTON, Philippe**
**F-57500 Saint-Avold (FR)**

(74) Mandataire:
**Rataboul, Michel Charles**
**CMR INTERNATIONAL,**
**10, rue de Florence**
**75008 Paris (FR)**

(56) Documents cités:
WO-A-93/04627                    US-A- 4 807 638

• **PROCEEDINGS OF THE 9TH ANNUAL CONFERENCE OF IEEE ENGINEERING IN MEDICINE AND BIOLOGY SOCIETY,, vol.3, 13 Novembre 1987, BOSTON, US, pages 1488 - 1489 MINGHAI QU ET AL, 'portable impedance cardiograph for ambulatory subjects'**
• **MEDICAL PROGRESS THROUGH TECHNOLOGY,, vol.19, 22 Avril 1993, BERLIN, DE, pages 105 - 113 S.HUNOLD ET AL 'computer supported analysis of cardiovascular parameters by impedance cardiography and plethysmography'**

**Description**

La présente invention concerne le domaine des examens médicaux, plus particulièrement le suivi médical sur des durées importantes et l'aide au diagnostic, et a pour objet un dispositif d'acquisition, de mesure et de traitement de signaux physiologiques ainsi qu'un procédé automatique mis en oeuvre par ledit dispositif.

Actuellement, le débit cardiaque et la pression ou tension artérielle sont mesurés par des appareils séparés et ce uniquement à des intervalles de temps importants et dans des conditions particulières, à savoir lors de consultations médicales ou d'examens par un personnel spécialisé.

En outre, la détermination de la tension artérielle est réalisée par des dispositifs mécaniques, nécessitant une compression et, par conséquent, non adaptés à une utilisation en continu.

De même le débit cardiaque, mesuré, de manière générale, par l'intermédiaire de méthodes invasives, nécessite un appareillage lourd et ne peut être déterminé en continu sur une durée importante.

Il existe également différents appareils mettant en oeuvre les principes de la variation de l'impédance d'un volume déterminé du corps humain et permettant de mesurer le débit cardiaque de manière non-invasive.

Toutefois, ces appareils sont du type fixe, non portatif, et ne permettent donc pas de déterminer le débit cardiaque d'un patient en vie courante, de manière continue.

En outre, ils font appel à un traitement, ou du moins à un prétraitement, analogique des signaux relevés, sujet à de nombreuses perturbations, et les formules de calcul mises en oeuvre ne reposent absolument pas sur des critères anatomiques ou physiologiques.

Par ailleurs, ces appareils connus ne permettent pas la détermination simultanée de la tension artérielle (moyenne, systolique ou diastolique) et ne réalisent pas un enregistrement des signaux relevés et/ou des paramètres calcules sur des durées importantes, en vue d'une exploitation globale ultérieure.

Enfin, ces appareils précités n'autorisent pas l'acquisition et l'enregistrement, par voie séparée et de manière simultanée, d'au moins un signal d'électrocardiogramme, qui permet de diversifier la provenance et la nature des signaux informants à traiter. Par exemple, on connaît les documents suivants :

- WO 93/04627 qui concerne le traitement du signal issu de la captation de signaux physiologiques, en vue d'améliorer les mesures d'impédance.
- US 4 807 638 qui décrit la mesure de la pression artérielle moyenne, au moyen de deux capteurs d'impédance.

La présente invention ne concerne pas le traitement électronique de signaux électriques mais un mode d'acquisition des signaux physiologiques au moyen d'un seul capteur qui mesure non pas une moyenne mais les variations d'une valeur caractéristique. La présente invention a notamment pour but de pallier l'ensemble des inconvénients précités.

A cet effet, elle a pour objet un dispositif pour l'acquisition, la mesure et le traitement de signaux physiologiques pour la détermination de paramètres cardiaques et circulatoires, caractérisé en ce qu'il comprend des moyens, d'une part d'acquisition, de mise en forme et de numérisation, sous forme de circuits intégrés d'un signal électrocardiographique et d'un signal représentant la variation de l'impédance d'un volume déterminé du corps d'un patient reliés à des électrodes disposées en des emplacements spécifiques sur ce dernier, d'autre part par une unité de prétraitement numérique coopérant avec des moyens de commande et d'affichage et un moyen de stockage amovible et/ou un moyen de transmission de l'ensemble des signaux numérisés ou de valeurs extraites de paramètres significatifs, pour chaque battement cardiaque, à partir desdits signaux numérisés et, enfin, par un dispositif central de traitement numérique recueillant et emmagasinant, par transferts successifs, les données issues du moyen de stockage ou du moyen de transmission et permettant l'analyse desdites données ou desdits paramètres sur des périodes temporelles importantes, leur comparaison avec des données ou des valeurs de paramètres antérieures et l'affichage et/ou l'impression de l'ensemble des données pouvant servir pour l'élaboration d'un diagnostic, les moyens d'acquisition étant disposés dans un premier boîtier relié aux électrodes et en ce que l'unité de prétraitement et les moyens de commande et d'affichage et de stockage et/ou le moyen de transmission sont regroupés dans un second boîtier, éventuellement attaché au poignet du patient, dispositif qui comprend en outre un dispositif central de traitement numérique, la liaison entre les deux boîtiers précités étant filaire ou par ondes hertziennes.

L'invention a également pour objet un procédé automatique pour la mesure et le traitement de signaux physiologiques pour la détermination de paramètres cardiaques et circulatoires, consistant, après différentes opérations d'initialisation, à relever en continu, au moyen d'électrodes au moins un signal électrocardiographique et un signal représentant la variation de l'impédance d'un volume déterminé du corps d'un patient, délimité par des électrodes émettrices et des électrodes réceptrices, à filtrer et à numériser ces signaux, puis à les traiter, caractérisé en ce qu'il consiste à déterminer notamment, pour chaque battement cardiaque, à partir de courbes correspondant respectivement au signal représentant la variation de l'impédance d'un volume défini du corps d'un patient, à la dérivée temporelle du signal précédent, et à la dérivée temporelle du signal électrocardiographique, la fréquence cardiaque, le débit cardiaque, le travail cardiaque et les pressions artérielles moyennes, systoliques et diastoliques, à afficher en temps réel la ou les valeurs d'un ou de plusieurs desdits paramètres, à mémoriser en continu l'ensemble des signaux numérisés, et enfin à transférer et à manipuler les données mémorisées.

L'invention sera mieux comprise grâce à la description ci-après, qui se rapporte à un mode de réalisation préféré, donné à titre d'exemple non limitatif, et expliqué avec référence aux dessins schématiques annexés, dans lesquels :

la figure 1 est un schéma synoptique de la partie portable de l'appareil conforme à l'invention, montrant également l'implantation des électrodes sur un patient;

la figure 2 est une vue en élévation laterale de la partie du corps d'un patient représentée à la figure 1.

les figures 3A, 3B et 3C représentent, sous forme de chronogrammes, respectivement le signal correspondant à la variation de l'impedance d'un volume défini du corps d'un patient, la derivée du signal précédent et la derivée du signal électrocardiographique.

la figure 4 représente, pour différents battements cardiaques successifs, les courbes d'évolution de la résistance périphérique indexée RV et du rapport IC;

la figure 5 est une vue en élévation latérale de supports d'électrodes, faisant partie d'un appareil selon l'invention;

la figure 6 représente la courbe C4: $dl/dt = F'(I)$ établie conformément au procédé selon l'invention;

la figure 7 représente la courbe C5: $(dl/dt)/I = F''(I)$ établie conformément a l'invention, et,

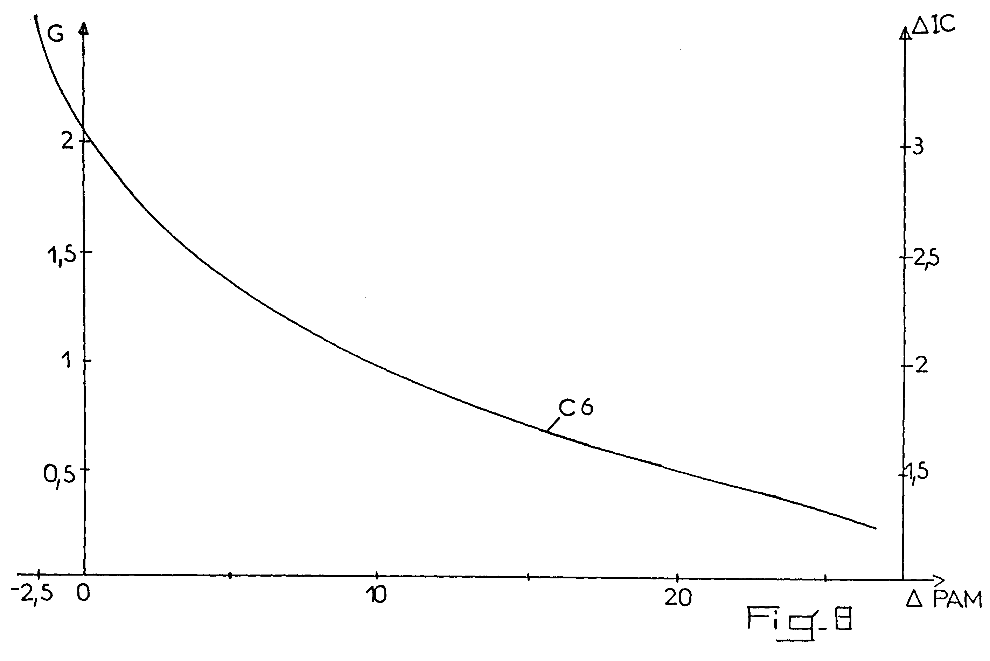

la figure 8 représente la courbe de régression expérimentale C6 établie selon l'invention.

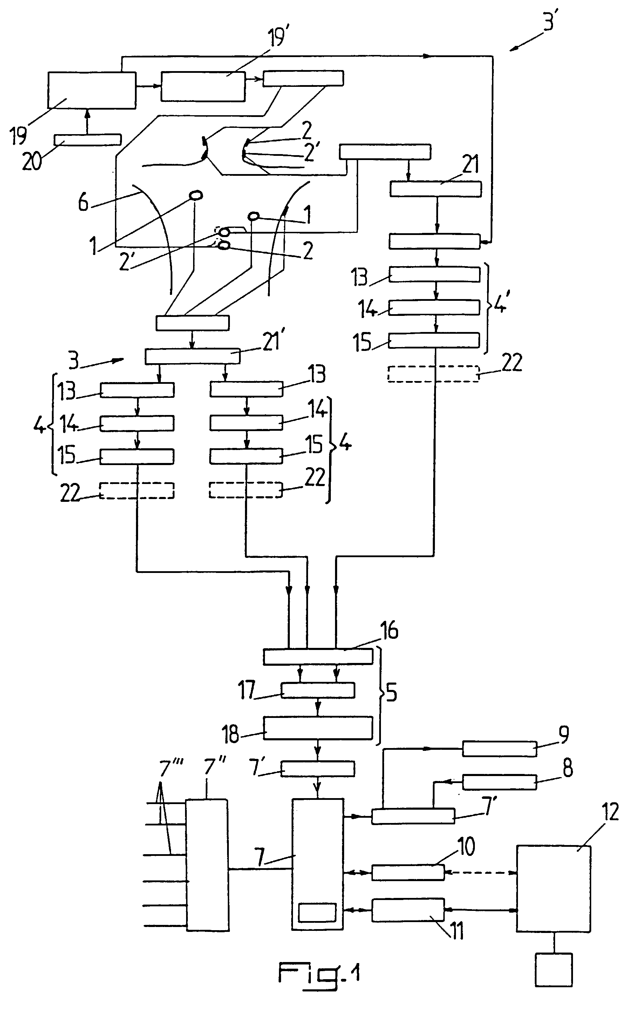

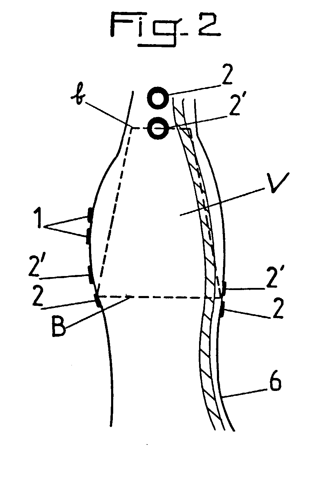

Conformément à l'invention, et comme le montrent les figures 1 et 2 des dessins annexés, l'appareil de mesure et de traitement est principalement constitué , d'une part, par des moyens d'acquisition 3, 3', de mise en forme 4, 4' et de numérisation 5 en continu d'au moins un signal électrocardiographique et d'un signal représentant la variation de l'impédance d'un volume V déterminé du corps 6 d'un patient, reliés à des électrodes 1, 2, 2' disposées en des emplacements spécifiques sur ce dernier, d'autre part, par une unité 7 de prétraitement numérique coopérant avec des moyens de commande 8 et d'affichage 9 et un moyen 10 de stockage amovible et/ou un moyen 11 de transmission de l'ensemble des signaux numérisés ou de valeurs extraites de paramètres FC, QC, WC, RV, IC, FE, PAM, PAS et PAD significatifs, pour chaque battement cardiaque, à partir desdits signaux numérisés et, enfin, par un dispositif central 12 de traitement numérique recueillant et emmagasinant, par transferts successifs, les données issues du moyen de stockage 10 ou du moyen de transmission 11 et permettant l'analyse desdites données ou desdits paramètres sur des périodes temporelles importantes, leur comparaison avec des données ou des valeurs de paramètres antérieures et l'affichage et/ou l'impression de l'ensemble des données pouvant servir pour l'élaboration d'un diagnostic.

Selon une première variante de réalisation de l'invention, les moyens 3, 3', 4, 4' et 5 d'acquisition, de mise en forme et de numérisation et l'unité de prétraitement 7 sont réalisés au moyen de circuits intégrés et regroupés, ensemble avec les moyens 8 et 9 de commande et d'affichage, dans un boitier portatif, de faible taille, éventuellement étanche et pourvu d'une alimentation autonome, le moyen de stockage (10) amovible consistant en une carte à mémoire enfichable à très grande capacité.

Cette disposition permet, par l'attribution d'un appareil de mesure à chaque patient, de relever et d'exploiter, en continu, le ou les signaux électrocardiographiques et le signal de variation d'impédance d'un patient, libre de ses mouvements, dans le cadre de sa vie et de ses activités courantes.

Ainsi, la mise en oeuvre de cet appareil ne s'accompagne d'aucune contrainte désagréable pour le patient (pas de compression, pas de limitation des mouvements quotidiens) et ne présente aucun risque d'utilisation (notamment mesure par technique non invasive).

En outre, les différents paramètres calculés FC, QC, WC, FE, PAM, PAD et PAS peuvent être affichés, en temps réel, au moyen, par exemple, d'un écran à cristaux liquides 9, ce en fonction du choix du patient porteur dudit boîtier ou d'une autre personne, transmis à l'unité de prétraitement numérique 7 par l'intermédiaire d'un clavier sensitif 8 par exemple. Ce dernier pourra également servir à l'initialisation de ladite unité de prétraitement numérique 7 notamment par la prise en compte de différents paramètres propres à chaque patient.

L'autonomie du boîtier portatif étant limitée, en priorité, par la capacité de mémorisation du moyen de stockage 10, il est avantageux de choisir une carte à mémoire du type connu sous la désignation "flash" et répondant à la norme connue sous la désignation PCM CIA, permettant de stocker, après une opération de compression des données, les signaux numérisés, les signaux obtenus après traitement ainsi que les paramètres calculés pour chaque battement cardiaque, ce sur une période d'au moins vingt-quatre heures.

Conformément à une seconde variante de réalisation avantageuse de l'appareil selon l'invention, non représentée aux dessins annexés, les moyens 3, 3', 4, 4' et 5 d'acquisition, de mise en forme et de numérisation, sous forme de circuits intégrés, sont disposés, dans un premier boîtier relié aux électrodes 1, 2, 2' et l'unité de prétraitement 7 et les moyens de commande et d'affichage 8 et 9 dont regroupés dans un second boîtier, éventuellement attaché au poignet du patient, la liaison entre les deux boîtiers précités étant du type filaire ou par ondes hertziennes.

Néanmoins, ledit boîtier pourra également être mis en oeuvre dans une version fixe indépendante ou intégrée dans un autre appareil de surveillance médical, notamment pour des applications cliniques par exemple, en étant alors alimenté directement par le secteur et relié au dispositif central 12 au moyen d'une liaison spécialisée du type RS232 ou d'une liaison série classique pour le transfert en continu des signaux numérisés et, le cas échéant, des valeurs des paramètres calculés pour chaque battement cardiaque.

Selon une autre caractéristique de l'invention, représentée à la figure 1 des dessins annexés, l'acquisition du signal représentant la variation d'impédance est réalisée au moyen d'un dispositif de détection synchrone 3' faisant circuler un courant électrique de faible intensité (1 mA) et de fréquence élevée réglable (40 KHZ - 120 KHZ), à travers un volume V défini, de forme tronconique, du tronc d'un patient et entre un ensemble d'électrodes émettrices 2 et un ensemble d'électrodes réceptrices 2' dont les emplacements délimitent ledit volume V.

Ledit dispositif 3' de détection synchrone, dont le principe est connu par l'homme de métier, produit à partir d'un circuit oscillateur 19 (sinus à très faible distorsion) pourvu d'un asservissement 20 (stabilisation + réglage de la fréquence de l'oscillateur 19), un signal haute fréquence généralement d'environ 75 KHz qui, converti par un circuit 19' adéquat en un signal de courant à faible intensité (de l'ordre de 1 mA), est appliqué au niveau des électrodes émettrices 2, puis recueilli, après transmission à travers le corps 6 du patient, au niveau des électrodes réceptrices 2'. Les signaux recueillis par ces dernières sont, ensuite, appliqués à un amplificateur différentiel 21 (à très haute impédance d'entrée) dont le signal de sortie est exploité, à l'aide du signal haute fréquence produit par l'oscillateur 19, en vue de l'extraction du signal représentant la variation de l'impédance du volume V défini par les électrodes 2 et 2'.

Les moyens d'acquisition 3 du signal électrocardiographique peuvent consister en un simple amplificateur différente 21'.

les moyens 4 de mise en forme du signal électrographique au moins relevé et les moyens 4' de mise en forme d'un signal de variation d'impédance présentent des structures semblables et sont constitués par des modules 13 de filtrage très sélectif (filtrage analogique 48 HZ/ordre 10) pour stabiliser les courbes relevées, par des modules 14 d'amplification et des modules 15 de calibration des signaux relevés.

Les moyens de numérisation 5 des signaux mis en forme comprennent , avantageusement, un dispositif d'échantillonnage/blocage 16, dont la sortie est reliée, par l'intermédiaire d'un circuit multiplexeur 17, à un circuit 18 de conversion analogique/digitale, préférentiellement sur 12 ou 16 bits.

Enfin, l'unité de prétraitement numérique 7 consiste préférentiellement en un microprocesseur 12 ou 16 bits muni de différents ports 7' d'entrée/sortie et d'une mémoire du type ROM contenant les différents programmes de gestion des différents moyens 3, 3', 4', 5, 8 et 9, de détermination des valeurs des paramètres et de stockage des données après compression.

Selon une autre caractéristique de l'invention, la fréquence d'émission et de mesure du dispositif de détection synchrone 3' est réglable numériquement à une valeur optimale pour un patient donné et les gains des moyens d'amplification des signaux 14, 21, faisant partie des moyens 4' de mise en forme du signal de variation d'impédance et/ou du dispositif 3' de détection synchrone, sont également réglables numériquement, par exemple par le dispositif central au cours d'une phase d'initialisation.

Comme le montre la figure 1 des dessins annexés, l'appareil de mesure selon l'invention peut comporter, en outre, un module d'interface 7'', pourvu de circuits de conversion analogique/digital et d'échantillonnage, permettant de fournir à l'unité de traitement 7, sous forme numérique, divers signaux délivrés par des capteurs supplémentaires fixés sur le patient, par l'intermédiaire de plusieurs lignes 7''' de connexion ou d'entrée de données.

Ainsi, il est possible de numériser et d'enregistrer simultanément plusieurs autres signaux donnant des indications sur l'état du patient et, immédiatement ou ultérieurement, de comparer en temps réel et sans aucun déphasage le signal d'ECG, de variation d'impédance et les différents signaux délivrés par les capteurs annexes.

Les signaux analogiques relevés par les capteurs annexes précités pourront par exemple consister en: signal de pression aortique, carotidogramme, signal piezzo-électrique, signal de dérivée première de la cinétique segmentaire ventriculaire gauche ou similaire, l'affichage synchrone de l'ensemble de ces signaux, en plus de ceux spécifiquement relevés par l'appareil transforme ce dernier en un véritable polygraphe portatif.

Les figures 1 et 2 illustrent un premier mode de réalisation de l'invention mettant en oeuvre quatre couples d'électrodes 2, 2' émettrice/réceptrice pour la mesure de la variation d'impédance d'un volume V d'un corps d'un patient, deux couples d'électrodes 2, 2' étant disposés au niveau du cou et les deux autres couples étant disposés au-dessus de la taille.

Selon un second mode de réalisation de l'invention, l'appareil comportera avantageusement seulement deux électrodes émettrices 2 et deux électrodes réceptrices 2', une première paire d'électrodes 2, 2' émettrice/réceptrice étant destinée à être appliquée sur la peau du patient au niveau de la partie supérieure du manubrium sternal et l'autre paire d'électrodes 2, 2', conjuguée à ou complémentaire de ladite première paire, étant destinée à être appliquée au niveau de l'appendice xyphoïde.

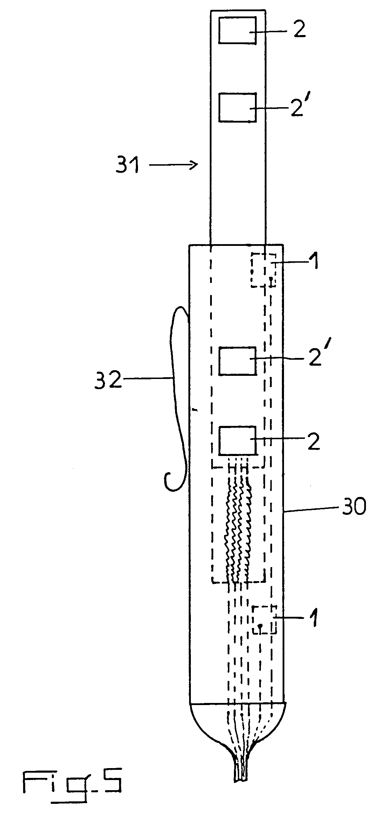

Conformément à une caractéristique de l'invention, représentée à la figure 5 des dessins annexés, les deux paires d'électrodes 2, 2' sont montées respectivement sur un support principal 30 et sur un support secondaire 31, de formes allongées et pouvant coulisser l'un par rapport a l'autre de manière à éloigner ou à rapprocher les deux paires d'électrodes 2, 2' l'une de l'autre, la distance entre les deux électrodes réceptrices 2' pouvant être déterminée visuellement au moyen d'une graduation.

Le support principal 30 peut avantageusement se présenter sous la forme d'un tube et porter, en plus d'une paire d'électrodes 2, 2', deux électrodes 1 d'acquisition de l'électrocardiogramme et un moyen d'accrochage 32 et le support secondaire 31 peut se présenter sous la forme d'une réglette ou d'un languette graduée, coulissable de manière télescopique par rapport au support principal 30 et portant une paire d'électrodes 2, 2' au niveau de son extrémité libre, la transmission entre lesdites électrodes 1, 2 et 2' et les moyens d'acquisition 3, 3' pouvant être réalisée par voie filaire ou par ondes infrarouges.

Les électrodes 1, 2 et 2' peuvent consister en des portions carrées de métal noble, d'environ 1 cm$^2$, serties sur les supports 30 et 31, les électrodes réceptrices 2' étant situées entre les deux électrodes émettrices 2 à environ 5 cm de ces dernières.

L'ensemble support principal 30/support secondaire 31 forme ainsi un dispositif compact et pouvant être transporté à la manière d'un gros stylo, en position escamotée, et manipulée par extension télescopique de la réglette 31 et application des électrodes 1, 2, 2' sur la peau du patient.

Comme le montre également la figure 1 des dessins annexés, un dispositif d'isolation optique 22 peut être disposé entre les moyens d'acquisition 3, 3' et de mise en forme 4, 4' des signaux, d'une part, et les moyens de numérisation 5 des signaux, d'autre part, permettant d'assurer une sécurité maximale du patient quel que soit le mode d'utilisation de l'appareil conforme à l'invention.

Toutefois, dans sa version portable, l'appareil conforme à l'invention étant alimenté au moyen de piles ou d'un accumulateur rechargeable, un tel dispositif d'isolation optique 22 est généralement inutile.

La réalisation pratique des différents dispositifs, modules ou circuit électroniques décrits ci-dessus, est connue de l'homme du métier et ne nécessite pas une description plus détaillée.

Il convient toutefois de noter que les divers signaux numériques sont codés sur au moins 12 bits ce qui permet d'obtenir une dynamique élevée.

L'invention a également pour objet un procédé automatique de mesure et de traitement mis en oeuvre par l'appareil décrit ci-dessus, consistant, après différentes opérations d'initialisation, à relever en continu, au moyen d'électrodes 1, 2 et 2' au moins un signal électrocardiographique et un signal représentant la variation de l'impédance d'un volume déterminé V du corps 6 d'un patient, délimité par des électrodes émettrices 2 et des électrodes réceptrices 2', à filtrer et à numériser ces signaux, puis à les traiter en calculant les signaux dérivés correspondants et en déterminant les valeurs, pour chaque battement cardiaque, de différents paramètres FC, QC, WC, RV, IC, FE, PAM, PAD, PAS cardiaques et circulatoires, à afficher en temps réel, en fonction du choix de l'utilisateur ou du patient, la ou les valeurs d'un ou de plusieurs desdits paramètres, à mémoriser en continu et pendant une période définie, l'ensemble des signaux numériques précités ainsi que les valeurs successives desdits paramètres sur un support 10 amovible et transportable ou dans une mémoire de grande capacité, et, enfin, à transférer et à manipuler les données mémorisées sur ledit support 10 ou dans ladite mémoire en vue, notamment, de leur analyse globale, de leur comparaison avec des données recueillies antérieurement et de leur édition sous forme de graphes ou de tableaux.

Selon une caractéristique de l'invention, les opérations d'acquisition, de filtrage, de numérisation, de traitement, d'affichage et de stockage sur un support 10 amovible sont réalisées par l'intermédiaire de moyens 3, 3', 4, 4', 5, 7 et 9 regroupés dans un boîtier portable à alimentation autonome, muni d'électrodes 1, 2 et 2' pour relever les signaux à traiter et à stocker.

La détermination du débit cardiaque QC est réalisée en exploitant par traitement numérique, différentes caractéristiques des courbes C1, C2 et C3 et en définissant, avec des critères anatomiques, le volume V dont la variation d'impédance est relevée.

Afin de permettre d'obtenir des informations autorisant un diagnostic efficace du patient concerné, le procédé selon l'invention consiste à déterminer notamment, pour chaque battement cardiaque, à partir des courbes C1, C2 et C3 correspondant respectivement, au signal I représentant la variation de l'impédance d'un volume V défini du corps 6 d'un patient, à la dérivée temporelle dI/dt du signal précédent, et à la dérivée temporelle du signal électrocardiographique, la fréquence cardiaque FC, le débit cardiaque QC, le travail cardiaque WC et les pressions artérielles moyennes PAM, systoliques PAS et diastoliques PAD.

Comme le montre la figure 2 des dessins annexés, le volume V délimité par les électrodes 2, 2' consiste en un tronc de cône dont la petite base b est située au niveau du cou du patient et dont la grande base B est située au niveau de la taille du patient, permettant de définir une constante K telle que:

$$K = H \times (D^2 + d^2 + D \times d - 3250)/60$$

avec

H = hauteur du tronc de cône en cm,

D = diamètre de la grande base en cm,

d = diamètre de la petite base en cm.

Toutefois, en mettant en oeuvre deux paires d'électrodes 2, 2' montés sur des supports 30 et 31, le volume V pris en compte pour la mesure de la variation d'impedance consiste en un tronc de cône situé entre le cou et le thorax au niveau scyphoïdien, permettant de définir une constante K telle que:

$$K = H \times (C^2 + C^2 + Cc - 2250)/60$$

avec

H = distance entre les deux électrodes réceptrices 2',

C = circonférence du thorax au niveau scyphoïdien,

c = circonférence du cou

Cette constante K est déterminée pour chaque patient et sa valeur est communiquée à l'unité de prétraitement numérique 7 au cours des opérations d'initialisation.

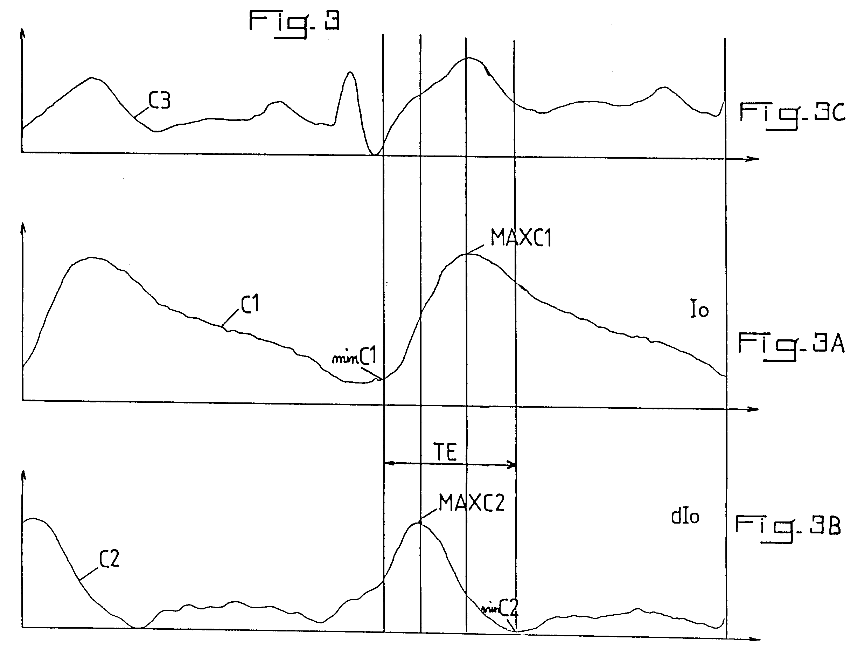

Conformément à l'invention, le procédé consiste, tout d'abord, a calculer, pour chaque battement cardiaque, la valeur du rapport IC = dlo/lo , où lo représente la différence entre la valeur maximale MAXC1 et la valeur minimale minC1 de la courbe C1, et dlo représente la différence entre la valeur maximalé MAXC2 et la valeur la plus fréquente de la courbe C2, ce pour chaque battement cardiaque (Figures 3A et 3B), les deux courbes C1 et C2 présentant des échelles d'ordonnées identiques.

Ensuite, en tenant compte de la valeur de IC calculée, comme indiqué précédemment; pour chaque battement cardiaque, et de la valeur de la constante K programmée avant le début du procédé, le procédé selon l'invention consiste à déterminer le volume éjecté systolique VES en litres et le débit cardiaque QC en litres/minute par les formules suivantes:

$$VES = K \times IC \times TEV \text{ et } QC = VES \times FC,$$

où TEV, temps d'éjection ventriculaire en s, est donnée par la formule:

$$TEV = H \times [(MINC2 - MAXC1) - (MAXC1 - MAXC2)],$$

avec H = 1,5 ± 0,15 , MINC2 étant la valeur minimale de la courbe C2 pour le battement cardiaque considéré.

La fréquence cardiaque FC exprimée en battements/minute est, de préférence, extraite de la dérivée du signal électrocardiographique, donc de la courbe C3, permettant ainsi de s'affranchir des parasitages affectant souvent le signal électrocardiographique (Figure 3C).

La fréquence cardiaque FC instantanée (en battements par minute) peut être calculée au moyen de la formule ci-après:

$$FC = (1000/R0R \ 1) \times 60$$

où R0R 1 représente l'écart temporel en millisecondes entre deux complexes consécutifs, cet écart pouvant être mesuré directement sur la courbe C3 de la figure 3.

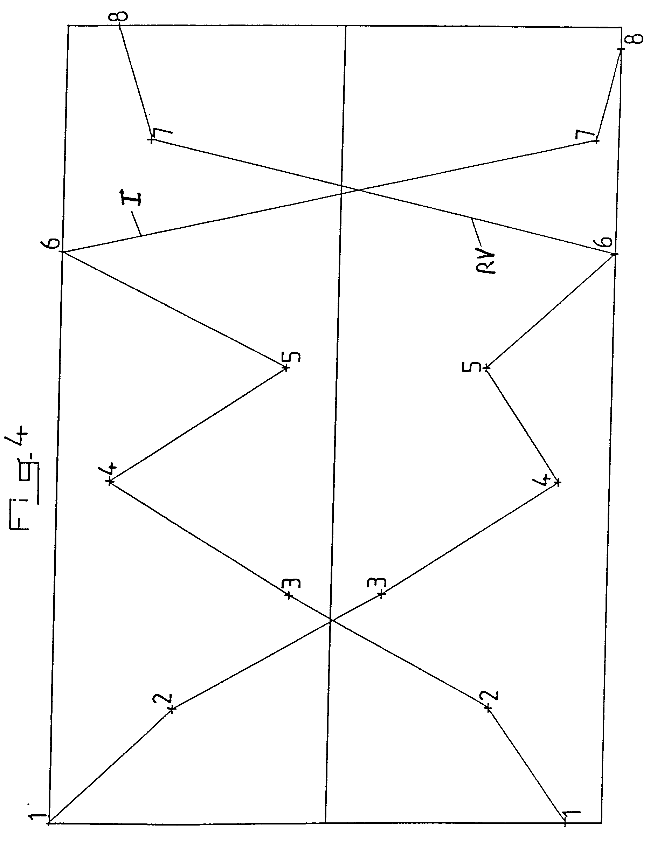

Au cours de différentes séries d'essais, il a été constanté, de manière surprenante, que la courbe représentant l'évolution du rapport IC et la courbe représentant l'évolution de la résistance périphérique indexée RV présentait des structures symétriques par rapport à un axe, les valeurs de IC et de RV pour chaque battement cardiaque, représentées sur un même graphe, étant disposées de part et d'autre d'un axe médian (Figure 4).

La présente invention utilise, avantageusement, cette particularité surprenante en vue de la détermination de la pression artérielle moyenne PAM.

A cet effet, le procédé conforme à l'invention consiste, à partir du rapport IC calculé pour chaque battement cardiaque, à déterminer la résistance périphérique indexée RV en dynes/cm$^5$/m$^2$ par l'intermédiaire de la formule suivante:

$$RV = \frac{(RV2 - RV1)(IC - IC1)}{IC2 - IC1}$$

où RV1 et RV2, respectivement IC1 et IC2, représentent des valeurs de RV, respectivement de IC, déterminées manuellement pour deux états différents du patient (au repos et à l'effort) et mémorisées lors des opérations d'initialisation.

De manière préférentielle, on relèvera et mémorisera plusieurs valeurs de RV et de IC préalablement à la mise en oeuvre du procédé selon l'invention, les valeurs RV1, RV2, IC1 et IC2 choisies, le cas échéant automatiquement au cours des opérations d'initialisation, étant celles vérifiant au mieux la règle de symétrie précitée.

La pression artérielle moyenne PAM peut alors être calculée , pour chaque battement cardiaque, par l'intermédiaire de la formule suivante:

$$PAM = \frac{RV \times QC}{80 \times SC}$$

où QC représente le débit cardiaque, RV la résistance périphérique indexée et SC la surface corporelle du patient soumis au procédé, la valeur du travail cardiaque WC étant alors déterminée par la formule:

$$WC = \frac{0{,}0144 \times PAM \times QC}{SC}$$

La valeur de la surface corporelle SC en cm$^2$, donnée par la formule [Taille (en cm) + Poids (en kg) - 60]/ 100 , sera également mémorisée, pour chaque patient, en tant que constante.

Conformément à une autre caractéristique de l'invention, la pression artérielle diastolique PAD peut ensuite être déterminée, pour chaque battement cardiaque, au moyen d'une fonction polynominale telle que:

$$PAD = F( X, Y)$$

avec $X = A/(RV - B)$ et $Y = C \times (WC - D)$ où A, B, C et D représentent des constantes, la fonction polynominale F( X, Y) présentant préférentiellement une formulation du type:

$$F(X, Y) = G \times (X + Y) - I \times (X + Y)^2 - J \times (X \times Y) + K + L,$$

où G, I, J, K et L représentent des constantes.

Les valeurs des constantes A, B, C, D, G, I, J, K et L peuvent être calculées en réalisant un grand nombre d'examens successifs au cours desquels il sera procéder à la détermination, de manière séparée, des valeurs de RV, de PAD, et de WC.

Néanmoins, à titre indicatif, ces différentes constantes pourront présenter les valeurs suivantes:

$$A \approx 0{,}0035,\ B \approx 2150,\ C \approx 1{,}8,\ D \approx 4{,}5,\ G \approx 6{,}4,\ I \approx 41,\ J \approx 165,\ K \approx 2140\ et\ L \approx 128.$$

A partir des valeurs de PAM et de PAD, il est alors aisé de déterminer la valeur de PAS, puisqu'il est connue que:

$$PAM = 1/3\ PAS + 2/3\ PAD$$

Suivant une autre caractéristique de l'invention, il est également possible de déterminer par calcul la fraction d'éjection FE par l'intermédiaire de la formule:

$$FE = -3{,}54 \times W + 60$$

à partir des paramètres:

$$D = \sqrt{\tfrac{1}{2} \times A^2 + \tfrac{1}{2} \times B^2 - AB}$$

où: $A = (RV - 2150)/450$ et
$B = (WC - 4{,}5)/0{,}9$
avec: $W = D$ si $A - B \geq 0$
et $W = -D$ si $A - B \leq 0$ .

Comme le montre notamment la figure 6 des dessins annexés, le procédé selon l'invention peut en outre, consister à établir numériquement, pour chaque battement cardiaque, une courbe C4 exprimant la fonction dl/dt = F'(I) et constituée par une première boucle ovoïde BO) représentant la systole mécanique et par une seconde boucle ovoïde bO représentant la diastole, puis à calculer le rapport entre les longueurs des grands axes des deux boucles BO et bO en vue d'éventuels calculs pression/volume ultérieurs.

En effet, le rapport dimensionnel axe BO/axe bO est, de l'avis des inventeurs, correlé avec le rapport volume éjecté auriculaire télédiastolique/volume ventriculaire télédiastolique et représente par conséquent un indice voisin de la compliance ventriculaire gauche.

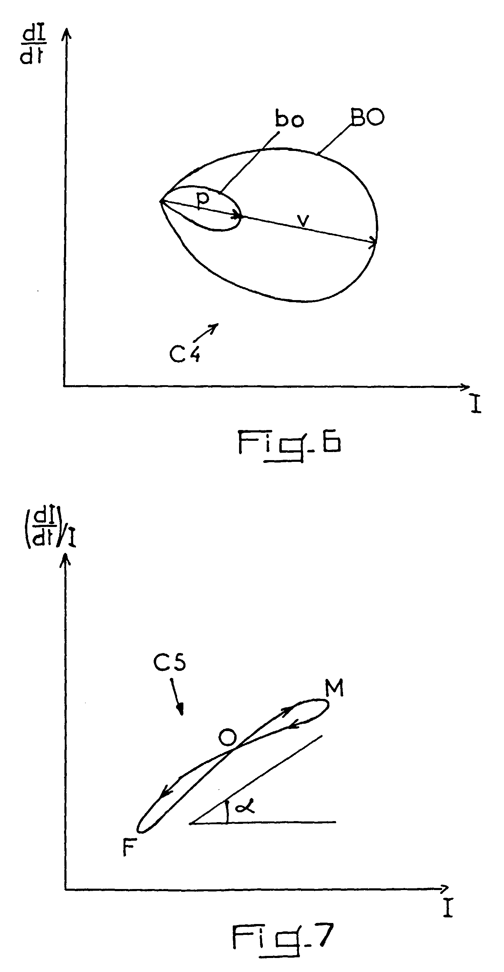

Selon une variante de réalisation de l'invention, illustrée par l'intermédiaire de la figure 7, des dessins annexés, le procédé peut également consister à établir numériquement, pour chaque battement cardiaque et à partir des valeurs numériques déterminées pour les courbes C1 et C2, une courbe C5 exprimant la fonction (dl/dt)/I = F"(I) , où I représente les valeurs successives relevées de l'impédance d'un volume V défini du corps du patient au cours d'un cycle cardiaque, et se présentant sous la forme d'un huit aplati dont l'axe longitudinal est incliné d'un angle $\alpha$ par rapport à l'axe correspondant à la variable 1, puis à mesurer ou à calculer le temps mis pour passer du point O d'intersection de la courbe C4 au point F situé à l'extrémité distale de la boucle inférieure de ladite courbe C4, ce temps correspondant au temps d'éjection ventriculaire TEV.

Conformément à une autre variante de réalisation de l'invention, le procédé peut consister à calculer un coefficient:

$G = (10^4 \times PFr \times 1,8)/(PAMr \times RVr \times Sc)$ où PFr, PAMr et RVr représentent respectivement les valeurs du rapport VES/TEV, de la pression artérielle moyenne et de la résistance périphérique indexée mesurées sur un patient à l'état de repos et SC la surface corporelle, puis à déterminer au moyen d'une courbe de régression C6, établie préalablement au moyen d'un grand nombre d'expériences et de mesures sur des patients différents, les valeurs incrémentales $\Delta$PAM et $\Delta$IC permettant, à partir des valeurs de PAM et IC mesurées ou calculées pour le patient considéré au repos, de calculer les valeurs maximales PAM max et IC max de PAM et IC par addition des valeurs incrémentales $\Delta$PAM et $\Delta$ IC aux valeurs au repos PAMr et ICr respectives, avec IC max = ICr x (1 + G/10) , à calculer ensuite RV à Nc max par l'intermédiaire de la formule:

PAM max = (QC max x RV à Nc max)/(80 x SC) où QC max représente le débit cardiaque maximale et Nc max représente la fréquence cardiaque maximale (en coups/minute) et, enfin, à déterminer, en utilisant la propriété de symétrie axiale des courbes représentant RV et IC, lesdites courbes RV et IC pour le patient considéré et à calculer les valeurs maximales de la pression artérielle diastolique PAD et de la pression artérielle systolique PAS.

La disposition décrite ci-dessus permet de s'affranchir, lors de la phase d'initialisation, de la réalisation d'une mesure des paramètres RV et IC notamment, pour un état d'effort du patient et autorise la mise en oeuvre dudit procédé par des partients à risques, alités, etc.

Ainsi il est possible, par une unique mesure effectuée à l'état de repos (avantageusement mesure moyennée pour une position debout et une position couchée) de déterminer la pression artérielle, le débit, la fraction d'éjection et le travail ventriculaire au maximum théorique des possibilités physiques d'un individu ou patient, ce qui peut être d'un grand intérêt pour la sélection et l'entraînement de sportifs.

Il en résulte également une possibilité d'appréciation des réserves d'un patient insuffisant cardiaque, hypertendu ou arythmique avant une anesthésie, avant ou après un traitement thérapeutique, avant ou après une intervention chirurgicale ou analogue.

En vue d'optimiser l'acquisition du signal de variation d'impédance en s'affranchissant au maximum des parasites, le procédé peut également consister à émettre, au cours des opérations d'initialisation, des signaux de test au niveau des électrodes émettrices 2, afin de déterminer notamment, par balayage fréquentiel, la fréquence d'émission permettant d'obtenir le signal le plus net à la réception au niveau des électrodes réceptrices 2'.

De manière avantageuse, la fréquence d'émission pour les mesures de variations d'impédance est réglée numériquement, en fonction d'une analyse comparative des signaux récupérés au niveau des antennes réceptrices 2' pour différents signaux d'émission de fréquences différentes.

En outre, pour tenter de ne prendre en considération que les signaux valides, le procédé selon l'invention consiste à effectuer, après traitement, un contrôle des signaux numérisés et des valeurs des paramètres déterminées pour chaque battement cardiaque, et à éliminer les signaux et les paramètres présentant des valeurs situées en dehors d'une plage prédéterminée, fixée lors des opérations d'initialisation.

A titre d'exemple, pour douze valeurs successives calculées pour les paramètres précités, on éliminera successivement celles situées à $\pm$ 50% de la moyenne desdites douze valeurs, puis celles situées à $\pm$ 30% de ladite moyenne.

Enfin, en vue d'augmenter la validité et la fiabilité de l'appareil et permettre une lecture aisée des résultats des mesures, ledit appareil peut avantageusement calculer et afficher les valeurs des paramètres FC, QC, WC, RV, IC, FE, PAM, PAS et PAD en établissant des moyennes sur dix battements cardiaques consécutifs.

Ainsi, l'appareil et le procédé conformes à l'invention permettent d'indiquer en permanence, en continu et en temps réel, la pression artérielle d'un patient, en synchronisme avec le signal d'ECG, un signal d'hémodynamisme et divers signaux de capteurs supplémentaires.

Bien entendu, l'invention n'est pas limitée aux modes de réalisation décrits et représentés aux dessins annexés. Des modifications restent possibles, notamment du point de vue de la constitution d'équivalents techniques, sans sortir pour autant du domaine de protection de l'invention.

## Revendications

1. Dispositif pour l'acquisition, la mesure et le traitement de signaux physiologiques pour la détermination de paramètres cardiaques et circulatoires, _caractérisé en ce qu_'il comprend des moyens, d'une part (3, 3', 4, 4' et 5) d'acquisition, de mise en forme et de numérisation, sous forme de circuits intégrés d'un signal électrocardiographique et d'un signal représentant la variation de l'impédance d'un volume (V) déterminé du corps (6) d'un patient reliés à des électrodes (1, 2, 2') disposées en des emplacements spécifiques sur ce dernier, d'autre part par une unité (7) de prétraitement numérique coopérant avec des moyens de commande (8) et d'affichage (9) et un moyen (16) de stockage amovible et/ou un moyen (11) de transmission de l'ensemble des signaux numérisés ou de valeurs extraites de paramètres (FC, QC, WC, RV, IC, FE, PAM, PAS et PAD) significatifs, pour chaque battement cardiaque, à partir desdits signaux numérisés et, enfin, par un dispositif central (12) de traitement numérique recueillant et emmagasinant, par transferts successifs, les données issues du moyen de stockage (10) ou du moyen de transmission (11) et permettant l'analyse desdites données ou desdits paramètres sur des périodes temporelles importantes, leur comparaison avec des données ou des valeurs de paramètres antérieures et l'affichage et/ou l'impression de l'ensemble des données pouvant servir pour l'élaboration d'un diagnostic, les moyens d'acquisition (3, 3', 4, 4' et 5) étant disposés dans un premier boîtier relié aux électrodes (1, 2, 2') et en ce que l'unité de prétraitement (7) et les moyens de commande et d'affichage (8 et 9) et de stockage (16) et/ou le moyen de transmission (11) sont regroupés dans un second boîtier, éventuellement attaché au poignet du patient, dispositif qui comprend en outre un dispositif central (12) de traitement numérique, la liaison entre les deux boîtiers précités étant filaire ou par ondes hertziennes.

2. Dispositif selon la revendication 1, _caractérisé en ce qu_'il comprend un moyen de détection synchrone (3') pour l'acquisition du signal représentant la variation d'impédance générant un courant électrique de faible intensité et de fréquence élevée réglable, circulant à travers un volume (V) défini de forme tronconique, du tronc d'un patient et entre un ensemble d'électrodes émettrices (2) et un ensemble d'électrodes réceptrices (2') dont les emplacements délimitent ledit volume (V).

3. Dispositif selon la revendication 2, _caractérisé en ce que_ la fréquence d'émission et de mesure du moyen de détection synchrone (3') est réglable numériquement à une valeur optimale pour un patient donné et en ce que les gains des moyens d'amplification des signaux (14, 21), faisant partie des moyens (4') de mise en forme du signal de variation d'impédance et/ou du dispositif (3') de détection synchrone, sont également réglables numériquement, par exemple par le dispositif central (12) au cours d'une phase d'initialisation.

4. Dispositif selon l'une quelconque des revendications 1 à 3, _caractérisé en ce qu_'il comporte un module d'interface (7"), pourvu de circuits de conversion analogique/digital et d'échantillonnage, permettant de fournir à l'unité de traitement (7), sous forme numérique, divers signaux délivrés par des capteurs supplémentaires fixés sur le patient.

5. Dispositif selon la revendication 1, _caractérisé en ce qu_'il comporte, pour la mesure de la variation d'impédance, deux électrodes émettrices (2) et deux électrodes réceptrices (2'), une première paire d'électrodes (2, 2') émettrice/réceptrice étant destinée à être appliquée sur la peau du patient au niveau de la partie supérieure du manubrium sternal et l'autre paire d'électrodes (2, 2'), conjuguée à ou complémentaire de ladite première paire, étant destinée à être appliquée au niveau de l'appendice xiphoïde.

6. Dispositif selon la revendication 5, _caractérisé en ce que_ les deux paires d'électrodes (2, 2') sont montées respectivement sur un support principal (30) et sur un support secondaire (31), de formes allongées et pouvant coulisser l'un par rapport à l'autre de manière à éloigner ou à rapprocher les deux paires d'électrodes (2, 2') l'une de l'autre, la distance entre les deux électrodes réceptrices (2') pouvant être déterminée visuellement au moyen d'une graduation.

7. Dispositif selon la revendication 6, _caractérisé en ce que_ le support principal (30) se présente sous la forme d'un tube et porte, en plus d'une paire d'électrodes (2, 2'), deux électrodes (1) d'acquisition de l'électrocardiogramme et un moyen d'accrochage (32) et en ce que le support secondaire (31) se présente sous la forme d'une réglette ou d'une languette graduée, coulissable de manière télescopique par rapport au support principal (30) et portant une paire d'électrodes (2, 2') au niveau de son extrémité libre, la transmission entre lesdites électrodes (1, 2 et 2') et les moyens d'acquisition (3, 3') pouvant être réalisée par voie filaire ou par ondes infrarouges.

8.  Dispositif selon la revendication 1, caractérisé en ce qu'un dispositif d'isolation optique (22) est disposé entre les moyens d'acquisition (3, 3') et de mise en forme (4, 4') des signaux, d'une part, et les moyens de numérisation (5) des signaux, d'autre part.

9.  Procédé automatique pour la mesure et le traitement de signaux physiologiques pour la détermination de paramètres cardiaques et circulatoires consistant, après différentes opérations d'initialisation, à relever en continu, au moyen d'électrodes (1, 2, 2') au moins un signal électrocardiographique et un signal représentant la variation de l'impédance d'un volume déterminé (V) du corps (6) d'un patient, délimité par des électrodes émettrices (2) et des électrodes réceptrices (2'), à filtrer et à numériser ces signaux, puis à les traiter, caractérisé en ce qu'il consiste à déterminer pour chaque battement cardiaque, à partir de courbes (C1, C2, C3) correspondant respectivement au signal I représentant la variation de l'impédance d'un volume (V) défini du corps (6) d'un patient, à la dérivée temporelle du signal précédent, et à la dérivée temporelle du signal électrocardiographique, la fréquence cardiaque (FC), le débit cardiaque (QC), le travail cardiaque (WC) et les pressions artérielles moyennes (PAM), systoliques (PAS) et diastoliques (PAD), à afficher en temps réel la ou les valeurs d'un ou de plusieurs desdits paramètres, à mémoriser en continu l'ensemble des signaux numérisés, et enfin à transférer et à manipuler les données mémorisées.

10. Procédé selon la revendication 9, caractérisé en ce qu'il consiste à calculer, pour chaque battement cardiaque, la valeur du rapport IC = dIo/Io , où (Io) représente la différence entre la valeur maximale (MAXC1) et la valeur minimale (minC1) de la courbe (C1), et (dIo) représente la différence entre la valeur maximale (MAXC2) et la valeur la plus fréquente de la courbe (C2), ce pour chaque battement cardiaque.

11. Procédé selon la revendication 9, caractérisé en ce que le volume (V) délimité par les électrodes (2, 2') consiste en un tronc de cône dont la petite base (b) est située au niveau du cou du patient et dont la grande base (B) est située au niveau de la taille du patient, permettant de définir une constante (K) telle que :

$$K = H \times (D^2 + d^2 + D \times d - 3250)/60$$

avec

$H$ = hauteur du tronc de cône en cm,
$D$ = diamètre de la grande base en cm,
$d$ = diamètre de la petite base en cm.

12. Procédé selon la revendication 9, caractérisé en ce que le volume (V) pris en compte pour la mesure de la variation d'impédance consiste en un tronc de cône situé entre le cou et le thorax au niveau scyphoïdien, permettant de définir une constante (K) telle que :

$$K = H \times (C^2 + C^2 + Cc - 2250)/60$$

avec

$H$ = distance entre les deux électrodes réceptrices 2',
$C$ = circonférence du thorax au niveau scyphoïdien,
$c$ = circonférence du cou.

13. Procédé selon la revendication 9, caractérisé en ce qu'il consiste à déterminer le volume éjecté systolique (VES) et le débit cardiaque (QC) par les formules suivantes :

$$VES = K \times IC \times TEV \text{ et } QC = VES \times FC,$$

où (TEV), temps d'éjection ventriculaire en s, est donnée par la formule :

$$TEV = H \times [(MINC2 - MAXC1) - (MAXC1 - MAXC2)],$$

avec H = 1,5 ± 0,15, (MINC2) étant la valeur minimale de la courbe (C2) pour le battement cardiaque considéré.

**14.** Procédé selon la revendication 9, <u>caractérisé en ce qu</u>'il consiste, à partir du rapport (IC) calculé pour chaque battement cardiaque, à déterminer la résistance périphérique indexée (RV) par l'intermédiaire de la formule suivante :

$$RV = \frac{(RV2 - RV1)(IC - IC1)}{IC2 - IC1}$$

où (RV1) et (RV2), respectivement (IC1) et (IC2), représentent des valeurs de (RV), respectivement de (IC), déterminées manuellement pour deux états différents du patient et mémorisées lors des opérations d'initialisation.

**15.** Procédé selon la revendication 9, <u>caractérisé en ce que</u> la pression artérielle moyenne (PAM) est calculée, pour chaque battement cardiaque, par l'intermédiaire de la formule suivante :

$$PAM = \frac{RV \times QC}{80 \times SC}$$

où (QC) représente le débit cardiaque, (RV) la résistance périphérique indexée et (SC) la surface corporelle du patient soumis au procédé, la valeur du travail cardiaque (WC) étant alors déterminée par la formule :

$$WC = \frac{0,0144 \times PAM \times QC}{SC}$$

**16.** Procédé selon la revendication 15, <u>caractérisé en ce que</u> la pression artérielle diastolique (PAD) est déterminée, pour chaque battement cardiaque, au moyen d'une fonction polynominale telle que :

$$PAD = F(X, Y)$$

avec X = A/(RV - B) et Y = C x (WC - D)
où A, B, C et D représentent des constantes.

**17.** Procédé selon la revendication 16, <u>caractérisé en ce que</u> la fonction polynominale F(X,Y) présente une formulation du type :

$$F(X,Y) = G \times (X + Y) - I \times (X + Y)^2 - J \times (X \times Y) + K + L,$$

où G, I, J, K et L représentent des constantes.

**18.** Procédé selon les revendications 14 et 15, <u>caractérisé en ce qu</u>'il consiste à calculer la fraction d'éjection (FE) par l'intermédiaire de la formule :

$$FE = -3,54 \times W + 60$$

à partir des paramètres :

$$D = \sqrt{\tfrac{1}{2} \times A^2 + \tfrac{1}{2} \times B^2 - AB}$$

où A = (RV - 2150)/450 et
B = (WC - 4,5)/0,9
avec W = D si A - B ≥ 0
et W = -D si A - B ≤ 0 .

**19.** Procédé selon la revendication 10, <u>caractérisé en ce qu</u>'il consiste à établir numériquement, pour chaque battement cardiaque, une courbe (C4) exprimant la fonction dI/dt = F'(I) et constituée par une première boucle ovoïde (BO) représentant la systole mécanique et par une seconde boucle ovoïde (bO) représentant la diastole, puis à calculer le rapport entre les longueurs des grands axes des deux boucles (BO et bO) en vue d'éventuels calculs pression/volume ultérieurs.

20. Procédé selon la revendication 9, <u>caractérisé en ce qu</u>'il consiste à établir numériquement, pour chaque battement cardiaque et à partir des valeurs numériques déterminées pour les courbes (C1 et C2), une courbe (C5) exprimant la fonction $(dI/dt)/I = F''(I)$ , où I représente les valeurs successives relevées de l'impédance d'un volume (V) défini du corps du patient au cours d'un cycle cardiaque, et se présentant sous la forme d'un huit aplati dont l'axe longitudinal est incliné d'un angle ($\alpha$) par rapport à l'axe correspondant à la variable (I), puis à mesurer ou à calculer le temps mis pour passer du point (O) d'intersection de la courbe (C4) au point (F) situé à l'extrémité distale de la boucle inférieure de ladite courbe (C4), ce temps correspondant au temps d'éjection ventriculaire (TEV).

21. Procédé selon la revendication 9, <u>caractérisé en ce qu</u>'il consiste à calculer un coefficient :
G = (10* x PFr x 1,8) / (PAMr x RVr x Sc)  où (PFr), (PAMr) et (RVr) représentent respectivement les valeurs du rapport VES/TEV, de la pression artérielle moyenne et de la résistance périphérique indexée, mesurées sur un patient à l'état de repos et (SC) la surface corporelle, puis à déterminer au moyen d'une courbe de régression (C6), établie préalablement au moyen d'un grand nombre d'expériences et de mesures sur des patients différents, les valeurs incrémentales ( PAM et IC) permettant, à partir des valeurs de PAM et IC mesurées ou calculées pour le patient considéré au repos, de calculer les valeurs maximales (PAM max. et IC max.) de PAM et IC par addition des valeurs incrémentales ( PAM et IC) aux valeurs au repos (PAM et ICr) respectives, avec IC max. = ICr x (1 + G/10) , à calculer ensuite RV à Nc max par l'intermédiaire de la formule :
PAM max = (QC max x RV à Nc max)/(80 x SC)  où (QC max) représente le débit cardiaque maximale et (Nc max) représente la fréquence cardiaque maximale en coups/minute et, enfin, à déterminer, en utilisant la propriété de symétrie axiale des courbes représentant (RV) et (IC), lesdites courbes (RV) et (IC) pour le patient considéré et à calculer les valeurs maximales de la pression artérielle diastolique (PAD) et de la pression artérielle systolique (PAS).

22. Procédé selon la revendication 9, <u>caractérisé en ce qu</u>'il consiste à émettre, au cours des opérations d'initialisation, des signaux de test au niveau des électrodes émettrices (2), afin de déterminer notamment, par balayage fréquentiel, la fréquence d'émission permettant d'obtenir le signal le plus net à la réception au niveau des électrodes réceptrices (2').

23. Procédé selon la revendication 22, <u>caractérisé en ce que</u> la fréquence d'émission pour les mesures de variations d'impédance est réglée numériquement, en fonction d'une analyse comparative des signaux récupérés, au niveau des antennes réceptives (2') pour différents signaux d'émission de fréquences différentes.

24. Procédé selon la revendication 9, <u>caractérisé en ce qu</u>'il consiste à effectuer, après traitement, un contrôle des signaux numérisés et des valeurs des paramètres déterminées pour chaque battement cardiaque, et à éliminer les signaux et les paramètres présentant des valeurs situées en dehors d'une plage prédéterminée, fixée lors des opérations d'initialisation.

25. Procédé selon la revendication 9, caractérisé en ce qu'il consiste à calculer et à afficher les valeurs des paramètres (FC, QC, WC, FE, PAM, PAS et PAD) en établissant des moyennes sur dix battements cardiaques consécutifs.

**Claims**

1. Device for the capture, measurement and processing of physiological signals for the determination of cardiac and circulatory parameters, <u>characterised in that</u> it comprises means, on the one hand (3, 3', 4, 4' and 5), for capturing, formatting and digitising, in the form of integrated circuits, an electrocardiographic signal and a signal representing the variation in impedance of a given volume (V) of the body (6) of a patient connected to electrodes (1, 2, 2') arranged at specific location on the latter, and on the other hand by a digital pre-processing unit (7) co-operating with control means (8) and display means (9) and a removable storage means (10) and/or and a transmission means (11) for all the digitised signals or values extracted from significant parameters (FC, QC, WC, RV, IC, FE, PAM, PAS and PAD), for each heartbeat, on the basis of said digitised signals and, finally, by a central digital processing device (12) collecting and storing, in successive transfers, the data coming from the storage means (10) or the transmission means (11) and permitting the analysis of said data or said parameters over considerable periods of time, their comparison with data or values of previous parameters and the display and/or printing of all the data that may serve for the development of a diagnosis, the capture means (3, 3', 4, 4' and 5) being arranged in a first housing connected to the electrodes (1, 2, 2') and in that the pre-processing unit (7) and the control and display means (8 and 9) and storage means (10) and/or the transmission means (11) are grouped together in a second housing, optionally attached to the wrist of the patient, which device further comprises a central device (12) for digital processing, the link between the two aforementioned housings being by wire or by radio waves.

2. Device according to Claim 1, <u>characterised in that</u> it comprises a synchronous detection means (3') for capturing the signal representing the variation in impedance generating an adjustable low-intensity, high-frequency electrical current, flowing through a defined volume (V), in the shape of a truncated cone, of the trunk of a patient and between a set of emitting electrodes (2) and a set of receiving electrodes (2'), the locations of which delimit said volume (V).

3. Device according to Claim 2, <u>characterised in that</u> the frequency of transmission and measurement of the synchronous detection means (3') is digitally adjustable to an optimum value for a given patient and in that the gains of the amplification means of the signals (14, 21), forming part of the means (4') for formatting the signal for variation in impedance and/or the synchronous detection device (3'), are also digitally adjustable, for example by the central device (12) during an initialisation phase.

4. Device according to any one of Claims 1 to 3, <u>characterised in that</u> it comprises an interface module (7"), provided with analogue-to-digital conversion and sampling circuits, making it possible to supply to the processing unit (7), in digital form, various signals delivered by additional sensors fixed to the patient.

5. Device according to Claim 1, <u>characterised in that</u> it comprises, for the measurement of the variation in impedance, two emitting electrodes (2) and two receiving electrodes (2'), a first pair of emitting/receiving electrodes (2, 2') being intended to be applied to the skin of the patient in the upper part of the manubrium sterni and the other pair of electrodes (2, 2'), combined with or in addition to said first pair, being intended to be applied to the xiphoid process.

6. Device according to Claim 5, <u>characterised in that</u> the two pairs of electrodes (2, 2') are mounted respectively on a main support (30) and on a secondary support (31), elongated in form and capable of sliding relative to one another so as to move the two pairs of electrodes (2, 2') further apart from each other or closer together, the distance between the two receiving electrodes (2') being capable of being visually determined by means of a graduation.

7. Device according to Claim 6, <u>characterised in that</u> the main support (30) takes the form of a tube and carries, in addition to a pair of electrodes (2, 2'), two capture electrodes (1) of the electrocardiogram and a hooking means (32) and in that the secondary support (31) takes the form of a rule or a graduated strip, slidable telescopically relative to the main support (30) and carrying a pair of electrodes (2, 2') at its free end, the transmission between said electrodes (1, 2 and 2') and the capture means (3, 3') being capable of being carried out by wire or by infrared waves.

8. Device according to Claim 1, <u>characterised in that</u> an optical isolation device (22) is arranged between the means for capturing (3, 3') and formatting (4, 4') the signals, on the one hand, and the means for digitising (5) the signals, on the other hand.

9. Automatic method for measuring and processing physiological signals in order to determine cardiac and circulatory parameters consisting, after various initialisation operations, in continuously picking up, by means of electrodes (1, 2, 2'), at least one electrocardiographic signal and a signal representing the variation in impedance of a given volume (V) of the body (6) of a patient, delimited by emitting electrodes (2) and receiving electrodes (2'), in filtering and digitising these signals, then in processing them, <u>characterised in that</u> it consists in determining, for each heart beat, on the basis of curves (C1, C2, C3) corresponding respectively to the signal I representing the variation in impedance of a defined volume (V) of the body (6) of a patient, to the time derivative of the preceding signal, and to the time derivative of the electrocardiographic signal, the cardiac frequency (FC), the cardiac output (QC), the cardiac work (WC) and the average arterial pressure (PAM), systolic pressure (PAS) and diastolic pressure (PAD), in displaying in real time the value or values of one or more of said parameters, in continuously storing all the digitised signals, and finally in transferring and manipulating the stored data.

10. Method according to Claim 9, <u>characterised in that</u> it consists in calculating, for each heart beat, the value of the relationship $IC = dIo/Io$, where (Io) represents the difference between the maximum value (MAXC1) and the minimum value (minC1) of the curve (C1), and (dIo) represents the difference between the maximum value (MAXC2) and the most frequent value of the curve (C2), for each heart beat.

11. Method according to Claim 9, <u>characterised in that</u> the volume (V) delimited by the electrodes (2, 2') consists in a truncated cone, the small base (b) of which is located level with the neck of the patient and the large base (B) of which is located level with the waist of the patient, making it possible to define a constant (K) such that:

$$K = H \times (D^2 + d^2 + D \times d - 3250) / 60$$

with

H = height of the truncated cone in cm,
D = diameter of the large base in cm,
d = diameter of the small base in cm.

12. Method according to Claim 9, <u>characterised in that</u> the volume (V) used for measuring the variation in impedance consists in a truncated cone located between the neck and thorax in the scyphoid area, allowing a constant (K) to be defined such that:

$$K = H \times (C^2 + C^2 + Cc - 2250)/60$$

With

H = distance between the two receiving electrodes 2',
C = circumference of the thorax in the scyphoid area,
c = circumference of neck.

13. Method according to Claim 9, <u>characterised in that</u> it consists in determining the systolic ejected volume (VES) and the cardiac output (QC) by the following formulae:

$$VES = K \times IC \times TEV \text{ and } QC = VES \times FC,$$

where (TEV), ventricular election time in s, is given by the formula:

$$TEV = H \times [(MINC2 - MAXC1) - (MAXC1 - MAXC2)],$$

with $H = 1.5 \pm 0.15$, (MINC2) being the minimum value of the curve (C2) for the heart beat in question.

14. Method according to Claim 9, <u>characterised in that</u>, on the basis of the ratio (IC) calculated for each heart beat, it consists in determining the indexed peripheral resistance (RV) through the following formula:

$$RV = \frac{(RV2 - RV1)(IC - IC1}{IC2 - IC1}$$

where (RV1 ) and (RV2), and respectively (IC1) and (IC2), represent values of (RV), (IC) respectively, determined manually for two different states of the patient and stored at the time of the initialisation operations.

15. Method according to Claim 9, <u>characterised in that</u> the average arterial pressure (PAM) is calculated, for each heart beat, through the following formula:

$$PAM = \frac{RV \times QC}{80 \times SC}$$

where (QC) represents the cardiac output, (RV) the indexed peripheral resistance and (SC) the surface area of the body of the patient undergoing the process, the value of cardiac work (WC) then being determined by the formula:

$$WC = \frac{0.0144 \times PAM \times QC}{SC}$$

**16.** Method according to Claim 15, <u>characterised in that</u> the diastolic arterial pressure (PAD) is determined, for each heart beat, using a polynomial function such that:

$$PAD = F (X, Y)$$

with $X = A / (RV - B)$ and $Y = C \times (WC - D)$
where A, B, C and D represent constants.

**17.** Method according to Claim 16, <u>characterised in that</u> the polynomial function F(X, Y) has a formulation of the type:

$$F(X, Y) = G \times (X + Y) - I \times (X + Y)^2 - J \times (X \times Y) + K + L,$$

where G, I, J, K and L represent constants.

**18.** Method according to Claims 14 and 15, <u>characterised in that</u> it

consists in calculating the ejection fraction (FE) using the formula:

FE = - 3.54 x W + 60 on the basis of the parameters:

$$D = \sqrt{\tfrac{1}{2} \times A^2 + \tfrac{1}{2} \times B^2 - AB}$$

where A = (RV - 2150) / 450 and
B = (WC - 4.5) / 0.9
with W = D if $A - B \geq 0$
and W = -D if $A - B \leq 0$.

**19.** Method according to Claim 10, <u>characterised in that</u> it consists in establishing numerically, for each heart beat, a curve (C4) expressing the function dI/dt = F'(I) and constituted by a first ovoid loop (BO) representing the mechanical systole and by a second ovoid loop (bO) representing the diastole, then in calculating the ratio between the lengths of the major axes of the two loops (BO and bO) with a view to optional subsequent calculations of pressure/volume.

**20.** Method according to Claim 9, <u>characterised in that</u> it consists in establishing numerically, for each heart beat and from numerical values determined for the curves (C1 and C2), a curve (C5) expressing the function (dI/dt)/I = F''(I) , where I represents the successive values read off for the impedance of a defined volume (V) of the body of the patient during a cardiac cycle, and taking the form of a flattened figure of eight, the longitudinal axis of which is inclined at an angle ($\alpha$) relative to the axis corresponding to the variable (I), then in measuring or calculating the time taken to move from the point (O) of intersection of the curve (C4) to the point (F) located at the distal end of the lower loop of said curve (C4), this time corresponding to the ventricular election time (TEV).

**21.** Method according to Claim 9, <u>characterised in that</u> it consists in calculating a coefficient: G = (10* x PFr x 1.8)/(PAMr x RVr x Sc) where (PFr), (PAMr) and (RVr) represent, respectively, the values of the relationship VES/TEV, the average arterial pressure and the indexed peripheral resistance, measured on a patient in the rest state and (SC) the surface area of the body, then in determining, using a regression curve (C6), established beforehand by means of a large number of experiments and measurements on different patients, the incremental values (PAM and IC) making it possible, using the values for PAM and IC measured or calculated for the patient in question at rest, to calculate the maximum values (PAM max. and IC max.) of PAM and IC by adding the incremental values (PAM and IC) to the respective values at rest (PAM and ICr), with IC max = ICr x (1 + G/10) , in then calculating RV at Nc max using the formula:
PAM max = (QC max x RV at Nc max) / (80 x SC) where (QC max) represents the maximum cardiac output and (Nc max) represents the maximum cardiac frequency in beats/minute and finally in determining, using the property of axial symmetry of the curves representing (RV) and (IC), said curves (RV) and (IC) for the patient in question and in calculating the maximum values for diastolic arterial pressure (PAD) and systolic arterial pressure (PAS).

**22.** Method according to Claim 9, <u>characterised in that</u> it consists in emitting, during the initialisation operations, test signals in the area of the emitting electrodes (2), in order to determine, in particular, by frequency scanning, the emission frequency making it possible to obtain the clearest signal on reception in the area of the receiving electrodes (2').

**23.** Method according to Claim 22, <u>characterised in that</u> the emission frequency for measurements of variations in impedance is governed numerically, as a function of a comparative analysis of the signals recovered, in the area of the receiving antennae (2') for different emission signals at different frequencies.

**24.** Method according to Claim 9, <u>characterised in that</u> it consists in making, after processing, a check on the digitised signals and the values of the parameters determined for each heart beat, and in eliminating the signals and parameters having values outside a predetermined range, fixed at the time of the initialisation operations.

**25.** Method according to Claim 9, characterised in that it consists in calculating and displaying the values of the parameters (FC, QC, WC, FE, PAM, PAS and PAD) by establishing averages over ten consecutive heart beats.

**Patentansprüche**

**1.** Vorrichtung zur Erfassung, Messung und Verarbeitung physiologischer Signale zur Bestimmung von Herz- und Kreislaufparametern, dadurch gekennzeichnet, daß sie einerseits Mittel (3, 3', 4, 4' und 5) zur Erfassung, Formung und Digitalisierung eines elektrokardiographischen Signals und eines die Impedanzveränderung eines bestimmten Volumens (V) des Körpers (6) eines Patienten darstellenden Signals in Form integrierter Schaltkreise, die mit Elektroden (1, 2, 2') verbunden sind, welche an spezifischen Stellen dieses Körpers angeordnet sind, andererseits eine Einheit (7) zur digitalen Vorverarbeitung, die mit Steuer- (8) und Anzeigemitteln (9) zusammenarbeitet, und ein abnehmbares Speichermittel (10) und/oder ein Mittel (11) zur Übertragung der Gesamtheit der digitalisierten Signale oder von aus diesen digitalisierten Signalen entnommenen Werten signifikanter Parameter (FC, QC, WC, RV, IC, FE, PAM, PAS und PAD) für jeden Herzschlag, und schließlich eine zentrale Vorrichtung (12) zur digitalen Verarbeitung aufweist, die durch aufeinanderfolgende Übertragungen die vom Speichermittel (10) oder vom Übertragungsmittel (11) kommenden Daten empfängt und speichert und die Analyse dieser Daten oder dieser Parameter über große Zeiträume, ihren Vergleich mit früheren Daten oder Parameterwerten und die Anzeige und/oder den Ausdruck der Gesamtheit der Daten ermöglicht, die zur Ausarbeitung einer Diagnose dienen können, wobei die Erfassungsmittel (3, 3', 4, 4' und 5) in einem ersten Gehäuse angeordnet sind, das mit den Elektroden (1, 2, 2') verbunden ist, und daß die Vorverarbeitungseinheit (7) und die Steuer- und Anzeigemittel (8 und 9) und die Speichermittel (10) und/oder das Übertragungsmittel (11) in einem zweiten Gehäuse zusammengefaßt sind, das ggf. am Handgelenk des Patienten befestigt ist, Vorrichtung, die außerdem eine zentrale Vorrichtung (12) zur digitalen Verarbeitung aufweist, wobei die Verbindung zwischen den beiden erwähnten Gehäusen eine Drahtverbindung oder eine Funkverbindung ist.

**2.** Vorrichtung nach Anspruch 1, dadurch gekennzeichnet, daß sie ein synchrones Erfassungsmittel (3') zur Erfassung des die Impedanzveränderung darstellenden Signals aufweist, das einen Schwachstrom mit einstellbarer, hoher Frequenz erzeugt, der durch ein definiertes, kegelstumpfförmiges Volumen (V) des Rumpfes eines Patienten und zwischen einer Einheit von Sendeelektroden (2) und einer Einheit von Empfangselektroden (2') fließt, deren Standorte das Volumen (V) begrenzen.

**3.** Vorrichtung nach Anspruch 2, dadurch gekennzeichnet, daß die Sende- und Meßfrequenz des synchronen Erfassungsmittels (3') auf einen für einen gegebenen Patienten optimalen Wert digital einstellbar ist und daß die Verstärkungen der Signal-Verstärkungsmittel (14, 21), die Teil der Mittel (4') zur Formung des Signals der Impedanzveränderung und/oder der synchronen Erfassungsvorrichtung (3') sind, ebenfalls digital einstellbar sind, zum Beispiel durch die zentrale Vorrichtung (12) während einer Initialisierungsphase.

**4.** Vorrichtung nach einem beliebigen der Ansprüche 1 bis 3, dadurch gekennzeichnet, daß sie einen Schnittstellenmodul (7'') aufweist, der mit Analog/Digital-Wandler- und Abtast-Schaltkreisen versehen ist und es ermöglicht, der Verarbeitungseinheit (7) in digitaler Form verschiedene Signale zu liefern, die von zusätzlichen, am Patienten befestigten Meßsonden abgegeben werden.

**5.** Vorrichtung nach Anspruch 1, dadurch gekennzeichnet, daß sie zur Messung der Impedanzveränderung zwei Sendeelektroden (2) und zwei Empfangselektroden (2') aufweist, wobei ein erstes Paar von Sende-/Empfangselektroden (2, 2') dazu bestimmt ist, auf die Haut des Patienten in Höhe des obersten Teils des Brustbeins aufgelegt zu werden, und das andere Paar von Elektroden (2, 2'), das dem ersten Paar zugeordnet oder dazu komplementär ist, in Höhe des Schwertfortsatzes angelegt wird.

6. Vorrichtung nach Anspruch 5, dadurch gekennzeichnet, daß die beiden Elektrodenpaare (2, 2') auf einem Hauptträger (30) bzw. einem Nebenträger (31) länglicher Form angeordnet sind, die in Bezug aufeinander gleiten können, um die beiden Elektrodenpaare (2, 2') voneinander zu entfernen oder einander anzunähern, wobei der Abstand zwischen den beiden Empfangselektroden (2') visuell mittels einer Gradeinteilung bestimmt werden kann.

7. Vorrichtung nach Anspruch 6, dadurch gekennzeichnet, daß der Hauptträger (30) in Form eines Rohrs vorliegt und zusätzlich zu einem Elektrodenpaar (2, 2') zwei Elektroden (1) zur Erfassung des Elektrokardiogramms und ein Befestigungsmittel (32) aufweist, und daß der Nebenträger (31) in Form einer mit Gradeinteilung versehenen Leiste oder Lasche vorliegt, die in Bezug auf den Hauptträger (30) teleskopisch gleiten kann und an ihrem freien Ende ein Elektrodenpaar (2, 2') trägt, wobei die Übertragung zwischen den Elektroden (1, 2 und 2') und den Erfassungsmitteln (3, 3') über eine Drahtverbindung oder über Infrarotwellen erfolgen kann.

8. Vorrichtung nach Anspruch 1, dadurch gekennzeichnet, daß eine Vorrichtung zur optischen Isolierung (22) zwischen den Erfassungsmitteln (3, 3') und den Formungsmitteln (4, 4') der Signale einerseits und den Digitalisierungsmitteln (5) der Signale andererseits angeordnet ist.

9. Automatisches Verfahren zur Messung und zur Verarbeitung physiologischer Signale zur Bestimmung von Herz- und Kreislaufparametern, das nach verschiedenen Initialisierungsvorgängen darin besteht, mittels Elektroden (1, 2, 2') mindestens ein elektrokardiographisches Signal und ein die Impedanzveränderung eines bestimmten, von Sendeelektroden (2) und Empfangselektroden (2') begrenzten Volumens (V) des Körpers (6) eines Patienten darstellendes Signal kontinuierlich zu erfassen, diese Signale zu filtern und zu digitalisieren und sie dann zu verarbeiten, dadurch gekennzeichnet, daß es darin besteht, für jeden Herzschlag ausgehend von Kurven (C1, C2, C3), die dem Signal I, das die Impedanzveränderung eines definierten Volumens (V) des Körpers (6) eines Patienten darstellt, bzw. der zeitlichen Ableitung des vorhergehenden Signals bzw. der zeitlichen Ableitung des elektrokardiographischen Signals entsprechen, die Herzfrequenz (FC), die Herzleistung (QC), die Herztätigkeit (WC) und die mittleren (PAM), systolischen (PAD) und diastolischen Arteriendrücke (PAD) zu bestimmen, in Echtzeit den Wert oder die Werte eines oder mehrerer dieser Parameter anzuzeigen, die Gesamtheit der digitalisierten Signale durchgehend zu speichern und schließlich die gespeicherten Daten zu übertragen und zu tasten,

10. Verfahren nach Anspruch 9, dadurch gekennzeichnet, daß es darin besteht, für jeden Herzschlag den Wert des Verhältnisses IC = dlo/lo zu berechnen, wobei (lo) den Unterschied zwischen dem Maximalwert (MAXC1) und dem Minimalwert (minC1) der Kurve (C1) und (dlo) den Unterschied zwischen dem Maximalwert (MAXC2) und dem häufigsten Wert der Kurve (C2) darstellt, und dies für jeden Herzschlag.

11. Verfahren nach Anspruch 9, dadurch gekennzeichnet, daß das von den Elektroden (2, 2') begrenzte Volumen (V) aus einem Kegelstumpf besteht, dessen kleine Basis (b) sich am Hals des Patienten und dessen große Basis (B) sich in Höhe der Taille des Patienten befindet, was es ermöglicht, eine Konstante (K) zu definieren, derart, daß :

$$K = H \times (D^2 + d^2 + D \times d - 3250)/60$$

mit

H = Höhe des Kegelstumpfs in cm,
D = Durchmesser der großen Basis in cm,
d = Durchmesser der kleinen Basis in cm.

12. Verfahren nach Anspruch 9, dadurch gekennzeichnet, daß das für die Messung der Impedanzveränderung berücksichtigte Volumen (V) aus einem Kegelstumpf besteht, der sich zwischen dem Hals und dem Brustkorb im schalenförmigen Bereich befindet, was es ermöglicht, eine Konstante (K) zu bestimmen, derart, daß:

$$K = H \times (C^2 + C^2 + Cc - 2250)/60$$

Mit

H = Abstand zwischen den beiden Empfangselektroden 2',
C = Umfang des Brustkorbs im schalenförmigen Bereich,
c = Umfang des Halses.

**13.** Verfahren nach Anspruch 9, dadurch gekennzeichnet, daß es darin besteht, das ausgestoßene systolische Volumen (VES) und die Herzleistung (QC) durch die folgenden Formeln zu bestimmen:

$$VES = K \times IC \times TEV \text{ und } QC = VES \times FC,$$

wobei (TEV), Ventrikel-Ausstoßzeit in s, durch die folgende Formel gegeben wird:

$$TEV = H \times [(MINC2 - MAXC1) - (MAXC1 - MAXC2)]$$

mit $H = 1{,}5 \pm 0{,}15$, wobei (MINC2) der minimale Wert der Kurve (C2) für den betrachteten Herzschlag ist.

**14.** Verfahren nach Anspruch 9, dadurch gekennzeichnet, daß es darin besteht, ausgehend vom für jeden Herzschlag berechneten Verhältnis (IC), den indexierten peripheren Widerstand (RV) mittels der folgenden Formel zu bestimmen:

$$RV = \frac{(RV2 - RV1)(IC - IC1)}{IC2 - IC1}$$

wobei (RV1) und RV2) bzw. (IC1) und (IC2) Werte von (RV) bzw. (IC) darstellen, die manuell für zwei unterschiedliche Zustände des Patienten bestimmt und bei den Initialisierungsvorgängen gespeichert werden.

**15.** Verfahren nach Anspruch 9, dadurch gekennzeichnet, daß der mittlere Arteriendruck (PAM) für jeden Herzschlag mittels der folgenden Formel berechnet wird:

$$PAM = \frac{RV \times QC}{80 \times SC}$$

wobei (QC) die Herzleistung, (RV) den indexierten peripheren Widerstand und (SC) die Körperoberflache des Patienten darstellt, die dem Verfahren unterworfen wird, wobei der Wert der Herztätigkeit (WC) dann durch die folgende Formel bestimmt wird:

$$WC = \frac{0{,}0144 \times PAM \times QC}{SC}$$

**16.** Verfahren nach Anspruch 15, dadurch gekennzeichnet, daß der diastolische Arteriendruck (PAD) für jeden Herzschlag mittels einer polynominalen Funktion bestimmt wird, derart, daß:

$$PAD = F(X, Y)$$

mit $X = A/(RV - B)$ und $Y = C \times (WC - D)$
wobei A, B, C und D Konstante darstellen.

**17.** Verfahren nach Anspruch 16, dadurch gekennzeichnet, daß die polynominale Funktion F(X, Y) eine Formulierung der Art

$$F(X, Y) = G \times (X + Y) - I \times (X + Y)^2 - J \times (X \times Y) + K + L$$

aufweist,

wobei G, I, J, K und L Konstante sind.

**18.** Verfahren nach den Ansprüchen 14 und 15, dadurch gekennzeichnet, daß es darin besteht, den Ausstoßanteil (FE) mittels der Formel

$$FE = 3{,}54 \times W + 60$$

zu berechnen, ausgehend von den Parametern:

$$D : \sqrt{\tfrac{1}{2} \times A^2 + \tfrac{1}{2} \times B^2 - AB}$$

wobei A = (RV - 2150)/450 und
B = (WC - 4,5)/0,9
mit W = D wenn A - B ≥ 0
und W = -D wenn A - B ≤ 0 ist.

19. Verfahren nach Anspruch 10, dadurch gekennzeichnet, daß es darin besteht, digital für jeden Herzschlag eine Kurve (C4) zu erstellen, die die Funktion dI/dt = F'(I) ausdrückt und aus einer ersten ovalen Schleife (BO), die die mechanische Systole darstellt, und aus einer zweiten ovalen Schleife (bO) besteht, die die Diastole darstellt, und dann das Verhältnis zwischen den Längen der großen Achsen der beiden Schleifen (BO und bO) für eventuelle spätere Druck/Volumen-Berechnungen zu berechnen.

20. Verfahren nach Anspruch 9, dadurch gekennzeichnet, daß es darin besteht, für jeden Herzschlag und ausgehend von bestimmten digitalen Werten für die Kurven (C1 und C2) digital eine Kurve (C5) zu erstellen, die die Funktion (dI/dt)/I = F''(I) ausdrückt, wobei I die erfaßten aufeinanderfolgenden Werte der Impedanz eines definierten Volumens (V) des Körpers des Patienten während eines Herzzyklus darstellt, und in Form einer abgeflachten Acht vorliegt, deren Längsachse um einen Winkel (α) in Bezug auf die der Variablen (I) entsprechende Achse geneigt ist, und dann die Zeit zu messen oder zu berechnen, die benötigt wird, um vom Schnittpunkt (O) der Kurve (C4) zum Punkt (F) zu gelangen, der sich am fernen Ende der unteren Schleife der Kurve (C4) befindet, wobei diese Zeiten der Ventrikel-Ausstoßzeit (TEV) entsprechen.

21. Verfahren nach Anspruch 9, dadurch gekennzeichnet, daß es darin besteht, einen Koeffizienten G = (10* x PFr x 1,8)/(PAMr x RVr x Sc) zu berechnen, wobei (PFr), (PAMr) und (RVr) die Werte des Verhältnisses VES/TEV bzw. des mittleren Arteriendrucks bzw. des indexierten peripheren Widerstands, gemessen an einem Patienten im Ruhezustand, und (SC) die Körperoberfläche darstellen, und dann mittels einer Regressionskurve (C6), die vorher mittels einer großen Anzahl von Erfahrungen und Messungen an verschiedenen Patienten erstellt wurde, die Inkrementwerte (PAM und IC) zu bestimmen, was es ermöglicht, ausgehend von den für den betreffenden Patienten im Ruhezustand gemessenen oder berechneten Werten von PAM und IC die maximalen Werte (PAM max. und IC max.) von PAM und IC durch Addition der Inkrementwerte (PAM und IC) zu den jeweiligen Werten im Ruhezustand (PAM und Icr) zu berechnen, mit IC max. = ICr x (1 + G/10) , dann RV bei Nc max mittels der Formel
PAM max = (QC max x RV bei Nc max)/(80 x SC) zu berechnen, wobei (QC max) die maximale Herzleistung und (Nc max) die maximale Herzfrequenz in Schläge/Minuten darstellt, und schließlich unter Verwendung der Eigenschaft der axialen Symmetrie der (RV) und (IC) darstellenden Kurven diese Kurven (RV) und (IC) für den betreffenden Patienten zu bestimmen und die maximalen Werte des diastolischen Arteriendrucks (PAD) und des systolischen Arteriendrucks (PAS) zu berechnen.

22. Verfahren nach Anspruch 9, dadurch gekennzeichnet, daß es darin besteht, während der Initialisierungsvorgänge Testsignale in Höhe der Sendeelektroden (2) auszugeben, um insbesondere durch Frequenzabtastung die Sendefrequenz zu bestimmen, die es ermöglicht, das klarste Signal beim Empfang in Höhe der Empfangselektroden (2') zu erhalten.

23. Verfahren nach Anspruch 22, dadurch gekennzeichnet, daß die Sendefrequenz für die Messungen von Impedanzveränderung in Abhängigkeit von einer vergleichenden Analyse der aufgefangenen Signale in Höhe der Empfangsantennen (2') für verschiedene Sendesignale verschiedener Frequenzen digital eingestellt wird.

24. Verfahren nach Anspruch 9, dadurch gekennzeichnet, daß es darin besteht, nach der Verarbeitung eine Kontrolle der digitalisierten Signale und der Werte der bestimmten Parameter für jeden Herzschlag durchzuführen und die Signale und die Parameter zu entfernen, die Werte aufweisen, welche sich außerhalb eines vorbestimmten Bereichs befinden, der bei den Initialisierungsvorgängen festgelegt wurde.

25. Verfahren nach Anspruch 9, dadurch gekennzeichnet, daß es darin besteht, die Werte der Parameter (FC, QC, WC, FE, PAM, PAS und PAD) zu berechnen und anzuzeigen, indem Mittelwerte von zehn aufeinanderfolgenden Herzschlägen erstellt werden.

Fig.1

Fig. 2

Fig.3

Fig.-3A    Io    MAXC1    minC1    C1

Fig.-3B    dIo    MAXC2    minC2    C2    TE

Fig.-3C    C3

Fig. 4

EP 0 710 086 B1

Fig.5

Fig. 6

Fig. 7

Fig_8